# EUROPEAN PATENT SPECIFICATION

(11) **EP 2 025 751 B1**
(45) Date of publication and mention of the grant of the patent: **17.11.2010**
(21) Application number: 08100507.6
(22) Date of filing: 15.01.2008
(51) Int. Cl.: C12N 15/10

(54) **Method for washing a column and method for extracting membrane-bound target molecules**
Verfahren zur Waschung einer Säule und Verfahren zur Extraktion membrangebundener Zielmoleküle
Procédé de lavage de colonne et procédé pour l'extraction de molécules cibles liées à la membrane

(30) Priority: 13.08.2007 TW 96129869
(43) Date of publication of application: 18.02.2009
(73) Proprietor: Taigen Bioscience Corporation, Taipei (TW)
(72) Inventor: Daf, David, Taipei (TW)
(74) Representative: 2K Patentanwälte Blasberg Kewitz & Reichel

(56) References cited:
- EP-A- 0 875 271
- EP-A- 1 529 840
- DE-A1- 19 746 874
- "QIAquick PCR purification kit; QIAquick nucleotide removal kit; QIAquick gel extraction kit" QIAQUICK SPIN TECHNOLOGY HANDBOOK, 1 November 2006 (2006-11-01), pages 1-44, XP002502339 Qiagen, Hilden, Germany

## Description

### Background of the Invention

### 1. Field of the Invention

The present invention relates to a method for washing a column and a method extracting membrane-bound target molecules such as silica membrane-bound nucleic acids, DNA, or RNA.

### 2. Description of the Related Art

A variety of commercial products adopt silica chaotropic technology-based membrane columns (or generally called spin columns) to isolate and purify nucleic acids (DNA/RNA). There are one or several membranes disposed at the bottom of a column, and the space within the column can be filled with liquid. When the liquid is loaded onto the membrane column, appropriate force is applied to draw the liquid through the bottom membrane, and subsequently the drawn liquid is discarded or collected. Generally, the so-called appropriate force used to draw the liquid through membranes may be centrifugal force, when the process is manipulated on a centrifuge, or atmospheric pressure, when vacuum manifold is adopted.

Generally, the purification procedure using silica membrane chaotropic technology comprises three steps, i.e., binding, washing, and eluting. The handbook for these commercial products usually provides two procedures for the operators' choice. One is centrifuge protocol, and the other is vacuum protocol. Compared to vacuum protocol, the centrifuge protocol comprises more steps and takes longer time, and therefore it is suitable for handling less number of samples. However, the centrifuge protocol is more stable because the liquid in the column passes through the membrane(s) more completely after each centrifugation procedure, and the residue of liquid scarcely remains in the column. On the other hand, the vacuum protocol is easier to operate and takes less time compared to the centrifuge protocol, and therefore it is suitable for handling a larger number of samples. However, the vacuum protocol is unstable because usually, the liquid in the column is unlikely to pass through the membrane(s) completely after each vacuum procedure, and there appears to be more liquid remaining in the column. In particular, at the end of the washing step, it is extremely likely that the residual washing liquid may contaminate the next eluting step and causes the eluted nucleic acid solution to contain the residual washing liquid. Consequently, such contaminant might affect the subsequent applications of the nucleic acids. Therefore, it is preferable to apply an additional centrifugation step after the washing step to eliminate the residual washing liquid. However, since the vacuum protocol can be easily manipulated at an automatic workstation, the additional centrifugation step will cause the whole procedure even more awkward.

Further, the washing liquid used in the silica chaotropic technology usually contain organic solvents such as alcohols. The main purpose of using washing liquid is to remove the undesired impurities without affecting the binding state of the nucleic acids and the membrane, while that of eluting buffers, usually water or low salt solution, is to elute the nucleic acids bound on the membrane so that they can be collected and used for the subsequent applications. Some of the applications of nucleic acids are sensitive to a small amount of organic solvents. For example, if the reagent used in the polymerase chain reaction (PCR) contains traces of alcohol, it will have noticeable inhibitory effects on the PCR. Alternatively, to eliminate the residual organic washing liquid in the membrane column, the membrane column can also be heated instead of centrifugation. But the heating condition must be adjusted to an optimal state. If the silica membrane is over-heated, it might become too dry, resulting in the bound nucleic acids' inability to be dissolved in the eluting buffer. Accordingly, the yield of nucleic acids might be reduced as a result. If the membrane is not heated enough, the residue of organic solvents cannot be eliminated.

Therefore, it is valuable to devise a method for washing a column and a method for extracting membrane-bound target molecules, such as silica membrane-bound nucleic acids, DNA, or RNA, for the application of vacuum protocol, especially for automation, to eliminate the residue of organic washing liquid, while no additional centrifugal step or heating step is necessary.

DE 197 46 874 A1 discloses a method and device for isolating and purifying nucleic acids on surfaces. Porous membranes are used to immobilize the nucleic acids from the sample containing the nucleic acids.

EP 1 529 840 A1 discloses a method for isolating nucleic acid from a nucleic acid source, wherein the nucleic acid source is lysed in the absence of a chaotropic salt and in the absence of an alcohol. The lysate is subsequently filtered through a porous matrix consisting of a material based on silica or of a silica coated material, which binds the nucleic acid in the absence of a chaotropic salt and in the absence of an alcohol. Finally, the nucleic acid is eluted from the porous matrix by an aqueous buffer solution.

### Summary of the Invention

It is an object of the present invention to provide an alternative washing step in a method of nucleic acid purification on a solid phase, in particular for washing a column for the application of vacuum protocol, especially for automation, to eliminate the residue of organic washing liquid and/or impurities.

This problem is solved by a method for washing a column according to claim 1 and by a method for extracting membrane-bound target molecules according to claim 4. Further advantageous embodiments are the subject-matter of the dependent claims.

According to the present invention, the method of washing a column is characterised in that the silica membrane column contains membrane-bound target molecules, such as nucleic acid, DNA or RNA, and impurities, and subsequently, the column is filled with a washing liquid such as eluting buffer or water having a capability of releasing the membrane-bound target molecules on the membrane to a liquid-free form, and finally, at least a portion of the washing liquid left in the column that does not pass through the membrane is subsequently removed from the top of the column so as to leave a clean membrane column for next elution step.

According to the other aspect of the present invention, a method for extracting membrane-bound target molecules containing the above washing method comprises the steps of:
a) preparing a column containing at least one membrane at its bottom end;
b) filling the column with a solution containing the membrane-bound target molecules and impurities;
c) drawing the solution through the membrane such that the membrane-bound target molecules are bound to the membrane and the solution is drained out of the column
d) filling the column with a first washing liquid to remove the impurities while still leaving the membrane-bound target molecules bound on the membrane;
e) drawing the first washing liquid through the membrane and out of the column;
f) filling the column with a second washing liquid having a capability of releasing the membrane-bound target molecules on the membrane to a liquid-free form;
g) removing the second washing liquid from the top of the column;
h) filling the column with an elution liquid so as to release the membrane-bound target molecules to a liquid-free form from the membrane; and
i) drawing the elution liquid through the membrane and collecting the elution liquid for the recovery of the membrane-bound target molecules.

Other objects, advantages and novel features of the present invention will be drawn from the following detailed description of preferred embodiment of the present invention with the accompanying drawings, in which:

### Descriptions of the Drawings

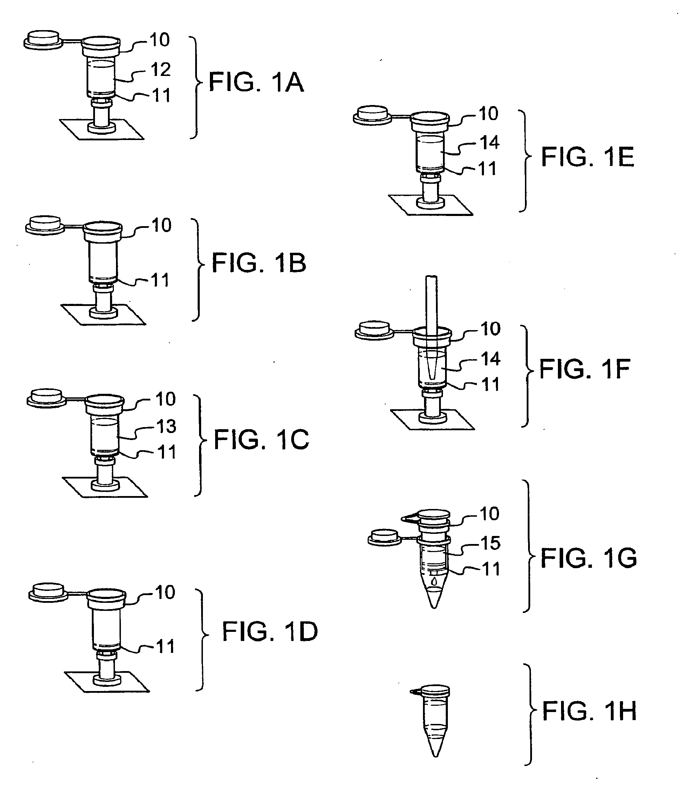

Fig. 1A illustrates a step of the method according to a preferred embodiment of the present invention;
Fig. 1B illustrates a step of the method according to a preferred embodiment of the present invention;
Fig. 1C illustrates a step of the method according to a preferred embodiment of the present invention;
Fig. 1D illustrates a step of the method according to a preferred embodiment of the present invention;
Fig. 1E illustrates a step of the method according to a preferred embodiment of the present invention;
Fig. IF illustrates a step of the method according to a preferred embodiment of the present invention;
Fig. 1G illustrates a step of the method according to a preferred embodiment of the present invention; and
Fig. 1H illustrates a step of the method according to a preferred embodiment of the present invention.

### Detailed Description of the Invention

A membrane in a column (or a spin column) generally is semi-permeable for liquid and a column has at least one membrane at its bottom end. When the column is filled with a liquid, the liquid can be blocked and usually retained for about one or several minutes. However, due to the influence of osmosis, the liquid can still go downwards and pass through the membrane slowly. Because of this phenomenon, when a liquid having a capability of releasing the membrane-bound target molecules from the membrane to a liquid-free form, is loaded into the column, the bound target molecules will be further brought into the membrane with the liquid pulled downwards by osmosis as soon as the liquid dissolves the bound target molecules to be a free form. This almost leaves no chance for the free target molecules to move upwards back to the liquid which is still retained in the column. Consequently, the target molecules and the liquid remained in the column are separated by the membrane and it can be assumed that the liquid in the column hardly contains the target molecules.

Accordingly, after a membrane column, such as a silica or glass membrane spin column, has been bound with target molecules, such as nucleic acid, DNA, or RNA and contains impurities, such as non-target molecules, for example, residuals of organic washing buffer or chaotropic salts after a washing step, a washing liquid having a capability of releasing the membrane-bound target molecules from the membrane to a liquid-free form, such as water or an elution buffer compatible with the subsequent applications of the target molecules, is loaded into the column, the target molecules will be further brought into the membrane with the washing liquid pulled downwards by osmosis as soon as the washing liquid dissolves the bound target molecules to a free form. Consequently, the target molecules and the liquid remained in the column are separated by the membrane. Accordingly, almost all of the target molecules are in or have passed through the membrane, while most of the impurities are still contained in the washing liquid and remained in the column. While at least a portion of the washing liquid in the column that does not pass through the membrane is removed, the impurities will be also removed together and almost none or a tiny amount of the target molecules are lost, which can subsequently effectively reduce the contaminations from the previous washing step (if any) for the subsequent eluting step.

Accordingly, a method for extracting membrane-bound target molecules containing the above washing method may comprise the steps of:
a) preparing a column 10 containing at least one membrane 11;
b) filling the column 10 with a solution 12 containing membrane-bound target molecules and impurities (referring to Fig. 1A);
c) drawing the solution 12 through the membrane 11 such that the membrane-bound target molecules are bound to the membrane 11 and the solution 12 is drained out of the column 10 (referring to Fig. 1B);
d) filling the column 10 with a first washing liquid 13, which is often organic, so as to remove the impurities while still leaving the target molecules bound on the membrane (referring to Fig. 1C);
e) drawing the first washing liquid 13 through the membrane 11 and out of the column 10 (referring to Fig. ID);
f) filling the column 10 with a second washing liquid 14, such as an eluting buffer or water, having the capability of releasing the membrane-bound target molecules from the membranes 11 to a liquid-free form (referring to Fig. 1E);
g) removing the second washing liquid 14 from the top of the column 10 (referring to Fig. IF);
h) filling the column 10 with an elution liquid 15 so as to release the target molecules to a liquid-free form from the membrane 11 (referring to Fig. 1G);
i) drawing the elution liquid 15 through the membrane 11 and collecting the elution liquid for the recovery of the membrane-bound target molecules (referring to Fig. 1H).

### Experiment:

Genomic DNA is purified using the QIAGEN QIAamp DNA Blood Mini kit according to the vacuum protocol. Briefly, a 1.7 ml blood sample in a 5 ml tube is mixed with 170 µl QIAGEN protease and 1.7 ml buffer AL, then, incubated at 56°C for 16 min, adding 1.7 ml ethanol to the sample, and mixed again by pulse-vortexing for 20 sec, then, aliquot 630 µl into 8 QIAamp spin columns, continuing the wash steps. At the end of the washing step, each spin column is weighted to approximately evaluate the amount of the residual washing liquid in the column; the average weight of spin column is increased to 25 mg +/- 2 mg as compared to their original weight.
Then, the 8 columns of samples are divided into 4 groups:
1. In Group I, 200 µl of eluting buffer (AE) is applied to the spin columns without further removing the residual washing buffer in the column, followed by a centrifugation step to elute the genomic DNA.
2. In Group II, an additional centrifugation step is included to remove the residual washing liquid in the column, then, the spin column is weighted again, about 20 micrograms of the residual washing liquid in the column were removed by centrifugation. Then, adding 200 µl of eluting buffer (AE), and the genomic DNA is eluted by the other centrifugation step.
3. In Group III, 850 µl of water is added into the spin column, and then is removed immediately. Finally, adding 200 µl of eluting buffer (AE) and eluting genomic DNA by centrifugation.
4. In Group IV, 850 µl of buffer (AE) is added into the spin column, and then is removed immediately. Finally, adding 200 µl of elute buffer (AE) and eluting genomic DNA by centrifugation.

The DNA quantity (O.D.260) of each sample is determined by the photo-spectrometer so as to evaluate the yield of nucleic acids. In addition, quantitative PCR (QPCR) using QuantiTect SYBR Green PCR kit (QIAGEN) is employed to evaluate the influence of different washing methods on QPCR by amplification and detection of GAPDH; 15 µl of eluate containing genomic DNA from each sample is mixed with 35 µl of QPCR master reagent containing primers. The results are listed in Table 1 below.

**Table 1**

| Group | Sample | Yield (OD260) | Ct of QPCR |
|---|---|---|---|
| I | 1 | 6.5 µg | 27.0 |
| | 2 | 6.8 µg | 27.5 |
| II | 3 | 6.6 µg | 22.8 |
| | 4 | 6.7 µg | 23.0 |
| III | 5 | 7.0 µg | 22.8 |
| | 6 | 6.4 µg | 23.0 |
| IV | 7 | 6.7 µg | 22.9 |
| | 8 | 6.6 µg | 22.9 |

From Table 1, it can be seen that the yields of four Groups are all close to each other, therefore, the method of the present invention does not reduce the yield of nucleic acid preparation, while adding and removing the additional washing liquid, such as eluting buffer AE or water, has the capability of releasing the nucleic acid from silica membrane. Further, analysis of the inhibition effect of the residual washing liquid in the columns from the previous washing step using QPCR shows that, Group I, without removing the residual washing liquid in the columns, has Ct values of about 27, whereas Group II has Ct values of about 23 using centrifugation to remove the residual washing liquid in the columns. The result obviously shows the inhibition effect of QPCR due to the co-elution of genomic DNA with the residual washing liquid in the columns. The Ct values of QPCR in Groups III and IV are all about 23 and close to the Ct values of Group II, showing that the method of the present invention can effectively remove the residual washing liquid in the columns from the previous washing step.

In conclusion, the present invention offers a method for washing a column and a method for extracting membrane-bound target molecules which are characterised by using an eluting buffer, water, or even any expected buffer as an additional washing liquid to effectively remove the impurities or residual washing liquid in the column from the previous washing step. The present invention can replace the aforementioned additional centrifugation or heating step between the traditional washing step and the eluting step without reducing the yield of target molecules (nucleic acids) for silica membrane chaotropic technology using a membrane column or a spin column, while it is so easy for operation, especially for automation.

While the invention has been described in terms of a preferred embodiment, those skilled in the art will recognise that the invention can still be practiced with modifications, within the spirit and scope of the appended claims.

## Claims

1. A method for washing a column (10) containing at least one membrane (11), impurities and membrane-bound target molecules, wherein
a washing liquid (14) having a capability of releasing the membrane-bound target molecules on the membrane to a liquid-free form is loaded to the column and
at least a portion of the washing liquid (14) left in the column that does not pass through the membrane is subsequently removed from the top of the column as to effectively remove the impurities in the column, in which method
the membrane (11) is a silica or glass membrane, and
the target molecules are nucleic acids, DNA, or RNA.

2. The method of any of claim 1, wherein the washing liquid is an elute buffer.

3. The method of claim 1, wherein the washing liquid is water.

4. A method for extracting membrane-bound target molecules comprising the steps of:
a) preparing a column (10) containing at least one membrane (11) at its bottom end;
b) filling the column with a solution (12) containing the membrane-bound target molecules and impurities;
c) drawing the solution through the membrane (11) such that the membrane-bound target molecules are bound to the membrane and the solution is drained out of the column
d) filling the column with a first washing liquid (13) to remove the impurities while still leaving the membrane-bound target molecules bound on the membrane;
e) drawing the first washing liquid (13) through the membrane (11) and out of the column (10);
f) filling the column with a second washing liquid (14) having a capability of releasing the membrane-bound target molecules on the membrane to a liquid-free form;
g) removing the second washing liquid (14) from the top of the column;
h) filling the column (10) with an elution liquid (15) so as to release the membrane-bound target molecules to a liquid-free form from the membrane; and
i) drawing the elution liquid (15) through the membrane (11) and collecting the elution liquid for the recovery of the membrane-bound target molecules;
in which method
the membrane (11) is silica or glass membrane,
the target molecules are nucleic acids, DNA, or RNA,
the first washing liquid (13) is organic, and
the second washing liquid (14) is an elute buffer or water.

## Patentansprüche

1. Verfahren zum Auswaschen einer Säule (10), die zumindest eine Membran (11), Verunreinigungen sowie membrangcbundcnc Zielmoleküle enthält, wobei eine Auswaschflüssigkeit (14) mit der Fähigkeit, die membrartgehundenen Zielmoleküle auf der Membran in einer flüssigkeitsfreien Form zu lösen, in die Säule eingegeben wird und
zumindest ein Teil der Auswaschflüssigkeit (14), die in der Säule zurückbleibt und nicht durch die Membran hindurch gelangt, anschließend von der Oberseite der Säule her entfcrnt wird, um so die Verunreinigungen in der Säule effizient zu entfernen, bei welchem Verfahren
die Membran (11) eine Siliziumdioxid- oder Glasmembran ist und
die Zielmoleküle Nukleinstiuren, DNA oder DNA sind.

2. Verfahren nach Anspruch 1, wobei die Auswaschflüssigkeit eine Eluierungs-Pufferflüssigkeit ist.

3. Verfahren nach Anspruch 1, wobei die Auswaschflüssigkeit Wasser ist.

4. Verfahren zum Extrahieren von membrangebundenen Zielmolekülen, mit den folgenden Schritten:
a) Vorbereiten einer Säule (10), die zumindest eine Membran (11) an ihrem unteren Ende aufweist;
b) Auffüllen der Säule mit einer Flüssigkeit (12), welche die membrangebundenen Zielmoleküle und Verunreinigungen enthält;
c) Absaugen der Flüssigkeit durch die Membran (11) hindurch, so dass die membrangebundenen Zielmoleküle an die Membran gebunden sind und die Flüssigkeit aus der Säule abgelassen ist;
d) Auffallen der Säule mit einer ersten Auswaschtltlssigkeit (13), um die Verunreinigungen zu entfernen, während die membrangebundenen Zielmolekule auch weiterhin auf der Membran gebunden verbleiben;
e) Absaugen der ersten Auswaschtlüssigkeit (13) durch die Membran (11) hindurch und aus der Säule (10) heraus;
f) Auffüllen der Säule mit einer zweiten Auswasehflüssigkeit (14) mit der Fähigkeit, die membrangebundenen Zielmoleküle auf der Membran in einer flüssigkeitsfreien Form abzulösen;
g) Entfernen der zweiten Auswaschflüssigkeit (14) vom oberen Ende der Säule her;
h) Auffüllen der Säule (10) mit einer Eluierungsflüssigkeit (15), um so die membrangebundenen Zielmoleküle in einer flüssigkeitsfreien Form von der Membran abzulösen;
i) Absaugen der Eluierungsflüssigkeit (15) durch die Membran (11) hindurch und Auffangen der Eluierungsflüssiglceit zur Rückgewinnung der membrangebundenen Zielmoleküle;
bei welchem Verfahren
die Membran (11) eine Siliziumdioxid- oder Glasmembran ist,
die Zielmoleküle Nukleinsäuren, DNA oder RNA sind,
die erste Auswaschflüssigkeit (13) organisch ist und
die zweite Auswaschflüssigkeit (14) eine Eluierungs-Pufferflüssigkeit oder Wasser ist.

## Revendications

1. Procédé de lavage d'une colonne (10) contenant au moins une membrane (11), des impuretés et des molécules cibles à liaison à une membrane, dans lequel
- un liquide de lavage (14) ayant une capacité de libération des molécules cibles à liaison à une membrane sur la membrane sous une forme exempte de liquide est chargé dans la colonne et
- au moins une partie du liquide de lavage (14) laissé dans la colonne qui ne traverse pas la membrane est ultérieurement retirée à partir du dessus de la colonne de façon à retirer de manière efficace les impuretés dans la colonne, dans lequel procédé
- la membrane (11) est une membrane de silice ou de verre, et
- les molécules cibles sont des acides nucléiques, de l'ADN ou de l'ARN.

2. Procédé selon la revendication 1, dans lequel le liquide de lavage est un tampon d'élution.

3. Procédé selon la revendication 1, dans lequel le liquide de lavage est de l'eau.

4. Procédé d'extraction de molécules cibles liées à la membrane comprenant les étapes consistant à :
a) préparer une colonne (10) contenant au moins une membrane (11) à son extrémité inférieure ;
b) remplir la colonne d'une solution (12) contenant des molécules cibles à liaison à une membrane et des impuretés ;
c) aspirer la solution à travers la membrane (11), de telle sorte que les molécules cibles à liaison à une membrane sont liées à la membrane et que la solution est drainée hors de la colonne ;
d) remplir la colonne d'un premier liquide de lavage (13) pour retirer les impuretés tout en laissant encore les molécules cibles à liaison à une membrane liées sur la membrane ;
e) aspirer le premier liquide de lavage (13) à travers la membrane (11) et hors de la colonne (10) ;
f) remplir la colonne d'un second liquide de lavage (14) ayant une capacité de libération des molécules cibles à liaison à une membrane sur la membrane sous une forme exempte de liquide ;
g) retirer le second liquide de lavage (14) à partir du dessus de la colonne ;
h) remplir la colonne (10) d'un liquide d'élution (15) de façon à libérer de la membrane les molécules cibles à liaison à une membrane sous une forme exempte de liquide ; et
i) aspirer le liquide d'élution (15) à travers la membrane (11) et collecter le liquide d'élution pour la récupération des molécules cibles à liaison à une membrane ;
dans lequel procédé
- la membrane (11) est une membrane de silice ou de verre,
- les molécules cibles sont des acides nucléiques, de l'ADN ou de l'ARN,
- le premier liquide de lavage (13) est organique, et
- le second liquide de lavage (14) est un tampon d'élution ou de l'eau.
